# EUROPÄISCHE PATENTSCHRIFT

(11) **EP 3 154 375 B1**
(45) Veröffentlichungstag und Bekanntmachung des Hinweises auf die Patenterteilung: **18.07.2018**
(21) Anmeldenummer: 15726571.1
(22) Anmeldetag: 27.05.2015
(51) Int. Cl.: A23L 11/00, B01D 11/02, C07C 51/42

(54) **VERFAHREN ZUR GEWINNUNG VON SINAPINSÄURE AUS EINEM NATIVEN STOFFGEMENGE**
METHOD FOR OBTAINING SINAPIC ACID FROM A NATIVE MATERIAL MIXTURE
PROCÉDÉ POUR L'OBTENTION D'ACIDE SINAPIQUE À PARTIR D'UN MÉLANGE DE MATIÈRES NATIVES

(30) Priorität: 28.05.2014 DE 102014107607
(43) Veröffentlichungstag der Anmeldung: 19.04.2017
(73) Patentinhaber: GEA Mechanical Equipment GmbH, 59302 Oelde (DE)
(72) Erfinder: HRUSCHKA, Steffen, 59302 Oelde (DE); ULLMANN, Detlef, 59302 Oelde (DE); BOSZULAK, Wladislawa, 59302 Oelde (DE); THIEL, Alexander, 67655 Kaiserslautern (DE)
(74) Vertreter: Specht, Peter
(86) Internationale Anmeldenummer: PCT/EP2015/061644
(87) Internationale Veröffentlichungsnummer: WO 2015/181203

(56) Entgegenhaltungen:
- DE-A1-102007 022 662
- DE-A1-102011 050 905
- EMBABY H E ET AL: "Glucosinolates and other anti-nutritive compounds in canola meals from varieties cultivated in Egypt and Japan", AFRICAN JOURNAL OF FOOD, AGRICULTURE, NUTRITION AND DEVELOPMENT, RURAL OUTREACH PROGRAM, KE , Bd. 10, Nr. 8 1. August 2010 (2010-08-01), Seiten 2967-2982, XP002688121, ISSN: 1684-5358 Gefunden im Internet: URL:http://www.ajfand.net/Volume10/No8/Emb aby7070.pdf [gefunden am 2012-10-18]

## Beschreibung

Die vorliegende Erfindung betrifft ein Verfahren zur Gewinnung von Sinapinsäure und/oder einem Salz der Sinapinsäure aus einem nativen Stoffgemenge.

Es ist bekannt, aus Saaten mit harten, zerbrechbaren Schalen, insbesondere aus Rapsfrüchten, eine Proteinphase als Wertstoffphase zu gewinnen.

Beim gängigen Ansatz zur Proteinkonzentratherstellung erfolgt eine Waschung der Schrote (stark entölt), wobei die löslichen Extraktionsstoffe abgereichert werden. Die Wertigkeit der entölten Zwischenprodukte hängt stark von der Konzentration an Begleitstoffen ab, wie Fasern, Zucker und sekundäre Pflanzenstoffen (Menner, M. u.a. "Fraktionierung pflanzlicher Rohstoffe zur simultanen Erzeugung von Lebensmitteln, technischen Rohstoffen und Energieträgern", Chemie Ingenieur Technik, Band 81, Ausgabe 11, Seiten 1743 - 1756, November 2009). Zu diesen Begleitstoffen zählen auch Polyphenole wie Sinapin. Die Polyphenolsäure "Sinapinsäure" kommt vor allem in Rapssamen vor (dort liegt der Sinapingehalt bei ca. 640 mg / 100 g Raps). Um Begleitstoffe wie Sinapin abzutrennen, werden große Verdünnungen gewählt, auch Proteine denaturiert (Temperatur, Alkohol), Zellulose wird enzymatisch abgebaut zu kurzkettigen Kohlenhydraten; diese Methoden werden gewählt, um die Stoffen besser extrahieren zu können.

Vor diesem Hintergrund ist es die Aufgabe der Erfindung, die Gewinnung von Wertstoffen aus dem nativen Stoffgemenge weiter zu optimieren, wobei es insbesondere möglich sein soll, auf relativ einfache Weise ein Sinapin, eine Sinapinsäure oder Derivate der Sinapinsäure zu gewinnen.

Die Erfindung löst diese Aufgabe durch die Merkmale des Anspruchs 1.

Weitere vorteilhafte Ausgestaltungen der Erfindung sind Gegenstand der Unteransprüche.

Das erfindungsgemäße Verfahren weist vorzugsweise folgende Schritte auf:

### Schritt A)

Als Ausgangsmaterial wird natives Stoffgemenge aus Saaten (Samen) mit harten, zerbrechbaren Schalen, insbesondere aus
- Saaten/Früchten von Kreuzblütengewächsen (Brassicaceae), insbesondere von Rapsfrüchten bereitgestellt.

Das Stoffgemenge im Sinne dieser Anmeldung kann aus den vollständigen, jedoch gebrochenen Saaten bestehen. Diese können ungeschält sein oder teilweise geschält oder ganz geschält.

Alternativ kann das Stoffgemenge aber auch aus einem bereits entölten Produkt bestehen, insbesondere aus einem "Zwischenprodukt", d.h. aus einem Presskuchen, der nach einer "Vorstufe", z.B. dem Abpressen von Öl, insbesondere mit einer Presse (z.B. einer Schneckenpresse) als Rückstand der Ölgewinnung verbleibt.

Besonders bevorzugt wird als das Ausgangsmaterial "kurz zuvor gewonnenes Zwischenprodukt" verarbeitet, d.h. nach der Vorstufe dürfen nicht mehr als 31 Tage vergangen sein.

Die Saat kann frisch geerntet oder aber Tage, Wochen oder Monate alt sein, die Zwischenstufe (das Pressen) sollte kurz oder sogar unmittelbar vor der weiteren Verarbeitung stattfinden, damit sich nach der Ölgewinnung das Material - die Saat - nicht zu stark verändert hat.

Ganz bevorzugt wird als das Ausgangsmaterial "frisches Material" verarbeitet, d.h. nach eine Vorstufe bzw. Vorbearbeitung (Ölgewinnen) dürfen nicht mehr als 3 Tage vergangen sein, vorzugsweise sogar nur weniger als 48 Stunden oder 24 Stunden oder 12 h oder weniger als 1 h.

Mit Material aus einem Zeitraum kurz nach der Vorstufe werden gute oder und mit frischem Material in der Regel nochmals bessere Ergebnisse hinsichtlich der Ausbeute und der Reinheit der Wertprodukte erzielt.

Der Presskuchen kann einen Restölgehalt aufweisen, der auch bei 20% oder mehr liegen kann. Trotz derart hoher Restölgehalte ist die Gewinnung auch einer Proteinphase mit der Erfindung auf einfache Weise realisierbar. Die Proteingewinnung ist dabei jedoch lediglich optional. Die Sinapinsäure und/oder das Sinapinsalz kann somit als einziges Wertprodukt des Stoffgemenges gewonnen werden oder als zusätzliches Wertprodukt bei einer Proteingewinnung gewonnen werden.

### Schritt B)

Sofern es noch nicht zerkleinert vorliegt: Zerkleinern des Stoffgemenges aus Schritt A) zum Aufbrechen der Schalen. Sofern ein Presskuchen verwendet wird, wird dieser aufgebrochen, idealerweise unmittelbar nach dem Pressen, noch warm. Derart wird ein zerkleinertes Material - eine Art Granulat - aus dem Presskuchen erzeugt. Das zuvor durch einen Pressvorgang (teil-) entölte Stoffgemenge wird in der Regel nur zerkleinert, beispielsweise zerbröselt bzw. granuliert oder es werden jedenfalls die Schalen aufgebrochen.

### Schritt C)

Das bereitgestellte und zerkleinerte Stoffgemenge aus Schritt A) oder B) wird mit Wasser oder einer wässrigen Lösung (z.B. einer Salzlösung) dispergiert. Auf einen Teil "zerkleinertes Produkt" werden vorzugswiese bis zu maximal 8, vorzugsweise bis zu maximal 5 Teile (Gewichtsanteile) Wasser zugegeben. Sodann werden Wasser und zerkleinertes Produkt gerührt, so dass sich ein fließfähiger Brei bzw. eine Dispersion ergibt. Das Rühren erfolgt vorzugsweise für mehr als 30 min, insbesondere für mehr als 1 h.

### Schritt D)

Als nächstes erfolgt ein Einstellen des pH-Wertes des Breis (I) aus Schritt C) in einen alkalischen Bereich pH > 9,5. Vorzugsweise wird der pH-Wert des Breis bzw. der Dispersion mit Lauge auf pH 10 bis 11 eingestellt. Dabei wird (vorsichtig) das Rühren fortgesetzt. Die Verrührzeit beträgt vorzugsweise mehr als 30 min, vorzugsweise liegt sie bei 1 h oder darüber.

### Schritt E)

In diesem weiteren Schritt erfolgt ein Zugeben mindestens eines wasserlöslichen organischen Lösemittels, vorzugsweise von Alkohol, insbesondere in mit Wasser verdünnter Form im Anschluss an das Einstellen des pH-Wertes des Breis im Schritt D. Vorzugsweise wird die Dispersion, deren pH-Wert in den alkalischen Bereich eingestellt worden ist, mit dem Alkohol EtOH (vorzugsweise 30-60%ig) auf eine Alkoholkonzentration von 20-15 Vol.% oder weniger, insbesondere 12% EtOH Konzentration gebracht. Entsprechend der Wassermenge des verwendeten Alkohols kann die Wassermenge in Schritt C um das im Alkohol, insbesondere im 30-60%igen EtOH enthaltene Wasser, reduziert werden. Damit lösen sich die Schalen vom Endosperm (Kotyledon) mit dem Restöl und können abgetrennt werden, insbesondere zentrifugal.

Als weniger bevorzugte Alternative gegenüber Ethanol können auch andere Alkohole, so z.B. Isopropanol, verwandt werden.

Die Schritte C-E können gemeinsam, beispielsweise zeitgleich, erfolgen. So ist es z.B. möglich eine wässrige stark verdünnte Ethanollösung mit Lauge auf einen entsprechend basischen pH-Wert einzustellen und diese Lösung dem zerkleinerten Stoffgemenge zuzugeben.

### Schritt F)

Im Schritt F) erfolgt daher ein Abtrennen einer Feststoffphase, welche den überwiegenden Anteil der Schalen umfasst, vorzugsweise in einer Zentrifuge im Zentrifugalfeld aus dem Brei bzw. es erfolgt ein Klären des Breis von Schalen-Feststoffanteilen insbesondere in einem Dekanter.

Bei diesem Schritt werden mit einem Dekanter mit einem Zulauf die Schalen von dem Restbrei getrennt.

Die leichtere Phase einer zentrifugalen Phasentrennung wird nachfolgend auch gelegentlich als Oberlauf bezeichnet und die Feststoffphase als schwere Phase. Eine Mittelphase läge entsprechend bzgl. ihrer Dichte dazwischen.

### Schritt G)

Der jedenfalls weitestgehend schalenfreie Brei aus Schritt F) wird nunmehr weiterverarbeitet. Vorzugsweise erfolgt dabei eine Ausfällung des gelösten - Proteinanteiles aus dem schalenfreien Brei, der zusammen mit dem un- oder angelösten Protein-Teil eine Fraktion bildet, den Quark. Der pH-Wert wird dabei wieder weiter in den sauren Bereich verschoben, in den pH-Bereich von pH = 4,5 bis pH = 7.

### Schritt H)

Sodann wird der schalenfreie Brei, dessen pH-Wert wieder ins Saure verschoben worden ist, - vorzugweise in einer Zentrifuge, insbesondere in wenigstens einem Dekanter oder in einem Separator - in einem oder zwei Schritten in Wertstoffphasen getrennt, von denen eine Phase eine Proteinkonzentratphase ist und eine dieser Phasen eine Polyphenol-Albumin-Flüssigkeitsphase ist;
Besonders bevorzugt erfolgt eine Trennung in folgende zwei oder drei Phasen:
- ölhaltige Phase
- wässrige Phase (Polyphenol- und Sinapinsäurehaltig)
- Proteinkonzentratphase (nachfolgenden auch "Proteinquark" genannt) oder
- wässrige Phase mit Albumin-, Polyphenolgehalt und Restölgehalt; und
- Proteinkonzentratphase (Proteinquark);
Die Zwei-Phasentrennung wird dann geführt, wenn das Rohmaterial relativ stark entölt ist und/oder im Feststoff gebunden vorliegt oder wenn kein intensiver Scherungseinfluss für die Flüssigkeitsphase in Schritt 1 ausgeführt worden ist. Die Zugabe von Wasser oder Alkohol oder Lauge oder dgl. kann auch in Teilschritten erfolgen. Das Öl als leichtere Phase enthält Triglyceride und ist einer der gewinnbaren Wertstoffe.
Schritt I) Isolieren von Sinapinsäure oder der Sinapines aus dem schalenfreien Brei des Schrittes F) oder - und dies wird besonders bevorzugt - aus der Polyphenol-Albumin-Flüssigkeitsphase des Schrittes H) direkt oder nach einem Durchlaufen weiterer Zwischenschritte, durch eine Extraktion mit einem Extraktionsmittel, insbesondere nach einem Zugeben eines Extraktionsmittels zum schalenfreien Brei aus Schritt F) oder G). Geeignet ist insbesondere Ethylacetat, was weiter unten anhand von Versuchen nachgewiesen wird. Einsetzbar ist aber auch Pentanol.
Nachfolgend wird zumeist von der Sinapinsäure und sinapinsäurehaltigen Phasen und Stoffgemengen gesprochen. Es versteht sich allerdings, dass je nach pH-Wert auch Sinapinsäure verestert mit Cholin als Sinapinsäure vorliegt.
Vorzugsweise liegt die Temperatur während sämtlicher Verfahrensschritte unter 60°C, insbesondere unter 50°C, vorzugsweise, zwischen 40 °C und 50°C, wodurch sich besonders wertvolle Produkte gewinnen lassen.
Die Denaturierung der Proteine ist ein temperatur- und zeitabhängiger Prozess. Hinzu kommt die Bedingung im alkoholischen Milieu. Die Proteindenaturierung geht umso schneller, je höher die Temperatur ist. In wässriger Umgebung ist aber auch bei Wärmeinwirkungen von 45-50 °C keine irreversible Proteindenaturierung zu erwarten. Das ändert sich aber mit der Alkoholkonzentration. Schon bei Umgebungstemperatur ist bei hochkonzentriertem Alkohol eine Proteinausfällung zu beobachten.
Je geringer nun die Alkoholkonzentration ist, umso höher muss die Temperatur sein, um die Proteine zu denaturieren. Oder umgekehrt: je wässriger die Alkoholkonzentration ist, umso höher darf die Arbeitstemperatur sein, ohne dass die Proteine irreversibel geschädigt werden.
Man wird also (für reines Wasser) eine möglichst hohe, d.h. möglichst an 60°C heranreichende Temperatur wählen, um möglichst viele Stoffe in Lösung zu bringen, wie Proteine, Lecithine, Glycolipide etc. Die gefällten Proteine liegen als Proteinquark vor (schwere Phase).
Die vorteilhafte Temperaturangabe zu den Verfahrensschritten A bis H bezieht sich nicht auf die Presstemperatur beim Erzeugen des Presskuchens bei der Ölerzeugung. Je höher die Temperatur bei den vorangegangen Prozessschritten war, umso brauner wird die Proteinphase bzw. Quarkfraktion. Dies liegt einerseits an der Maillard-Reaktion von Zuckern mit Proteinen, andererseits und der Phenoloxidation. Gegenüber der DE 10 2011 050 905 A1 wird insbesondere durch die Verwendung optimiert ausgewählten Ausgangsmaterials (vorzugsweise kalt gepresster Raps-Presskuchen, vorzugsweise sehr frisch) ein besonders ansprechendes, besonders gut weiterverwertbares Produkt gewonnen.

Besonders vorteilhaft ist die Verwendung kalt gepressten Materials (insbesondere eines kalt gepressten Rapspresskuchens (Temperatur beim Pressen vorteilhaft kleiner als 70°C, besonders vorzugsweise sogar kleiner als 60°C) als Ausgangsmaterial bzw. als das bereitgestellte Stoffgemenge. Warm gepresstes Material wird beim Pressen deutlichen höheren Temperaturen (bis über 100° C) ausgesetzt. Durch die Verwendung kalt gepressten Materials als Ausgangsmaterial für das erfindungsgemäße Verfahren kann eine Proteinphase bzw. "Protein- bzw. Quarkphase" mit deutlich besseren Eigenschaften (insbesondere hinsichtlich der Farbe deutlich heller und daher besser verarbeitbar) und mit deutlich besserer Ausbeute gewonnen werden als bei der Verwendung warm bzw. heiß gepressten Ausgangsmaterials. Dies wurde im Stand der Technik bisher nicht erkannt. Denn gängige Rapspressverfahren zielen auf eine hohe Ölausbeute, weshalb beim Pressen gern höhere Temperaturen verwendet werden. Als Nebeneffekt ist festzustellen, dass Sinapin (ein Polyphenol) abgebaut wird, was an sich vorteilhaft für die Proteinfraktion wäre. Beim erfindungsgemäßen Verfahren stellt der originale, also nicht reduzierte, Sinapingehalt im kalt gepressten Kuchen aber dennoch kein Problem für das Endprodukt dar, da die polyphenolischen Verbindungen im Wesentlichen nicht in der Quarkphase zu finden sind, da sie in die Wasserphase übergehen.

Dabei ist die Quarkphase, die nach dem erfindungsgemäßen Verfahren aus einem zuvor mit Hexan zusätzlich entöltem Presskuchen gewonnen wurde eher dem RAL-Ton 1024 ockergelb oder 1014 Elfenbein oder einem Gemisch dieser beiden Farben zuzuordnen. Die Verarbeitung erfolgt vorzugsweise unter Umgebungsdruck.

Auch in der Flüssigkeitsphase bzw. "Wasserphase" des Schrittes H) sind noch wertvolle Inhaltsstoffe enthalten, insbesondere ist sie relativ stark albuminhaltig. Sinnvoll und vorteilhaft ist insofern eine Filtration der Wasserphase zur Albumin-Aufkonzentrierung, um derart die Albuminphase als weiteren Wertstoff zu gewinnen.
Als weiterer wertvoller Inhaltstoff lassen sich aus der Flüssigkeitsphase bzw. "Wasserphase" des Schrittes H) Polyphenole gewinnen. Die Sinapinsäure wird mit einem dazu geeigneten Extraktionsmittel aus der wässrigen Phase extrahiert. Als Extraktionsmittel eignen sich insbesondere Ethylacetat und Pentanol, wobei dem Ethylacetat der Vorzug gegeben wird.
Alternativ ist es denkbar, die Extraktion der Sinapinsäure mit einem dazu geeigneten Extraktionsmittel direkt aus der Flüssigkeitsphase - dem proteinhaltigen Brei - des Schrittes F) vorzunehmen.
Eine besonders vorteilhafte Verfahrensvariante sei anhand des folgenden Beispiels erläutert.
Schritt A) Das bereitgestellte Ausgangsmaterial ist bei diesem Beispiel gepresster Rapskuchen, idealerweise schonend und kalt gepresst, mit typischen Restölgehalten von 20%; auch höher stellt kein Problem dar).
Schritt B) Der Kuchen wird aufgebrochen, idealerweise unmittelbar nach dem Pressen, noch warm.
Schritt C) Das Kuchengranulat wird mit Wasser dispergiert (1 Teil Kuchen und max. 6 Teile Wasser) und vorsichtig zu rühren (1 h).
Schritt D) Diese Dispersion ist mit Lauge auf pH 10 bis 11 einzustellen und vorsichtig zu rühren, üblicherweise 1 h.
Schritt E) Die Dispersion aus D ist mit EtOH (vorzugsweise 30-60%ig - bezogen auf Volumenprozent) auf 12 Vol.% EtOH Konzentration zu bringen, somit wird die Wassermenge in Punkt 3. um das in diesem 30-60%igen EtOH enthaltene Wasser reduziert.
Schritt F) Damit lösen sich die Schalen vom Endosperm (Kotyledon) mit dem Restöl und können zentrifugal abgetrennt werden.

Schritt G) Ausfällung vom Protein durch Ansäuern auf pH = 4,5 bis 7,2 aus dem Oberlauf (Oberlauf: Leichte Phase der Separation aus Schritt F mit einem pH-Wert von vorzugsweise 9,7 bis 10,5) zur Trennung: Öl - Wässrige Phase - Proteinkonzentratphase (Proteinquark) oder Trennung in Öl/Wasserphase und Proteinkonzentratphase; Unterstützt werden kann dieser Schritt durch einer intensive Scherung, um die Ölfreisetzung zu erleichtern.
Schritt H) Abtrennung der gefällten Proteine als Quark (schwere Phase (in der Regel Feststoffphase bzw. hier Quarkphase)) und ggf. Triglyceride (als leichtes Öl) aus dem Oberlauf (leichte Phase), insbesondere zentrifugal.
Schritt I) Filtration der Wasserphase zur Albumin-Aufkonzentrierung.
Als besonders vorteilhaft zu erwähnen ist die Nasstrennung der Schalen von den gelösten und gequollenen Proteinen bei parallel stattfindender Verdrängungsextraktion der Triglyceride (Ölphase) aus Öl- oder restölhaltigem Presskuchen oder Leguminosen Mehl und parallel verlaufender Polyphenolextraktion.
Die besonderen Vorteile des Verfahrens sind:
Es sind geringe Verdünnungen und damit geringe Volumenströme im Prozess durch das vorbeschriebene Verfahren realisierbar bei gleichzeitig geringem Lösungsmittelabfall.

Es ergibt sich eine höhere Polyphenolkonzentrationen während der Extraktion in der wässrigen Phase (Verfahrensschritte B bis D),

Native temperaturempfindliche Proteine sind zudem in einem weiteren Endprodukt enthalten, da der Prozess bei max. 50-55°C oder weniger umgesetzt wird.
Es sind insgesamt vergleichsweise hohe Proteinausbeuten mit bis zu 75 Gew.% erzielbar, wobei bis zu 45-50 Gew.% aus der "Quarkphase" und ca. 22-24 Gew.% aus der Albuminphase gewonnen werden können.

Es kann ein höherwertiges Proteingemisch gewonnen werden, weil Schalenreste, sowie Polyphenole, Kohlenhydrate, Lignin, Zellulose, etc. vollständig entfernt oder abgereichert sind.
Die Proteinphase enthält "natives" Protein, dessen quellfähiger Anteil nach der Gewinnung quellfähig bleibt und dessen wässrig lösbarere Anteile nach der Gewinnung wasserlöslich bleiben. Die Proteinphase ist zudem nahezu triglyceridfrei und weist nur geringe Restölwerte auf, hauptsächlich Polarlipide.
Das gute Milieu für Mikroorganismenwachstum durch die leichte Alkoholkonzentration vereinfacht die Prozesshygiene.
Der Alkohol kann verdünnt im Kreislauf gefahren werden.
Zu den Schritten A) und B):
Anstelle der Extraktion unerwünschter Stoffe aus dem stark entölten, feinst zerkleinerten Ausgangsmaterial Rapsschrot oder Rapskuchen -wie bei den gängigen Verfahren üblich-, erfolgt hier zunächst eine Abtrennung der Schalen im Nassen.
Dies wird in einem mehrstufigen Prozess dadurch gelöst, dass zunächst der Kuchen aufgebrochen wird, ohne die Kernbruchstücke noch weiter zu zerkleinern. Insbesondere kommt es darauf an, die Schalen möglichst groß zu belassen. Vorzugsweise sollten sie einen mittleren Durchmesser von 0,5 mm oder mehr aufweisen. Öltröpfchen brauchen nicht größer sein, wichtig sind nicht einzelne Moleküle oder kleine Molekülverbände, sondern "Partikel".
Dann wird Wasser zugegeben und im Alkalischen wird vorsichtig gerührt. Damit wird der wasserlösliche Teil der Proteine gelöst, ein anderer Teil quillt. Die Zugabe von wässrigem Alkohol verdrängt das freie Triglycerid als spezifisch leichte Phase aus der Dispersion. Die Lecithine, insbesondere Phosphatidylcholine, sind bei geringen Alkoholkonzentrationen löslich (siehe EP 1272048 B1 und zugehörige Patentfamilie). In dieser Kombination Lauge-wässriger Alkohol sind die zwei oder drei Phasen 1) Schwer = Schalen und 2) Leicht = Protein-Lecithin-Polyphenol- Kohlenhydrat zusammen mit ölhaltigem Schaum; oder
1) Schwer = Schalen, 2) Mitte = Protein-Lecithin-Polyphenol- Kohlenhydrat 3) Leicht = Triglycerid, vorteilhaft trennbar, so im Versuch im Becherglas oder im industriellen Maßstab vorzugsweise zentrifugal.
Je besser die Abtrennung der Schalen gelingt, umso geringer sind die Proteinverluste und umso reiner ist ein Endprodukt. Selbst die durch Wasserzugabe auf das bis zu 7 fache gequollene Schale ist schwerer als die Proteine in der alkoholisch-wässrigen Dispersion. Dies ist essentiell für eine Trennung durch Gravitation. Erschwert wird die Trennung aber durch ein festes Kleben der proteinhaltigen Aleuron Körper(Wabenschicht) an den Schalen. Diese Zellen sind dickwandig. Da die Zellmembran fast aller Zellen Lecithine enthält (neben Proteinen und anderen), kann nun durch geeignete Maßnahmen das Kleben durch ein "In-Lösung-Bringen" der Lecithine minimiert werden.
Konkret wird dies dadurch erreicht, dass die wässrige Phase eine Alkoholkonzentration von 5-40 Vol.% aufweist. (siehe Schritte S2-S4), idealerweise 12% bis 20%.
Entscheidend dafür ist bereits die Qualität des Ausgangsmaterials. Gewöhnlich ist bei kalt gepresstem Kuchen der Restölgehalt höher. Dies stört bei dem hier dargestellten Verfahren nicht. Im Gegenteil: Äußerst hilfreich ist die schönende Pressung, je moderater die Presstemperatur und desto geringer ist der Pressdruck, umso leichter ist eine nachfolgende Trennung von Schalen und Kotyledon (Keimblätter, das Kerninnere).

Das Verfahren ist auch mit "gewöhnlichem" d.h. heiß gepresstem Presskuchen anwendbar. Nur werden hierbei die Ausbeuten an Proteinen entsprechend geringer.

Zu den Schritten C) bis E) (Dispersionsherstellung):
Die Dispersionsherstellung mit Wasser- Lauge und Alkohol hat zwei Ziele: Einerseits das Lösen von der Schale, andererseits das Extrahieren phenolischer Verbindungen wie Sinapin aus dem Rohmaterial. Dabei ist die Benetzung mit Fluid wichtig. Jedoch erzeugte ein Scheren bei der der Dispersionsformulierung in den Schritten 2-5 Kleinstpartikeln, die zur Verunreinigungen in den separierten Phasen führten. Ohne eine Scherkopfmischer-Anwendung bzw., ohne die Verwendung einer Zahnscheibenmischers für die Schritte 2-5 betrug der Proteingehalt in der Proteinphase (nach dem Gewinnungsschritt und einer Trocknung) : >60 Gew.% bei frischem Material.

Unter Verwendung eines Scherkopfmischers bzw. mit Anwendung eines Frisam-Schermischers betrug der Proteingehalt in der Proteinphase (dem Gewinnungsschritt und einer Trocknung): ca. 50% bei altem Material

Ein anderer Test wurde mit heiß gepresstem Expeller-Kuchen durchgeführt. Die Menge an abtrennbaren Schalen in der ersten Stufe wird durch das Scheren von 20% auf 16% vermindert, gleichzeitig erhöht sich die Menge an ausfällbarem Protein aus der Klarphase von 38 Gew.% auf 42 Gew.%. Die Reinheit bleibt mit 39-40 Gew.% relative konstant und niedrig.

Die Wasser- Lauge und Alkohol Dispersion wird bei einer Temperatur von T = 50°C etwa 30 min gerührt.

Neben der Lösung der Lecithine im Wässrige-Alkoholischen hat zur verbesserten Abtrennung von Schalen einerseits und Triglyceriden anderseits hat die Abtrennung im leicht Alkoholischen zusätzlich den Vorteil, dass ein Wachstum von Mikroorganismen im Prozess erschwert wird. Dies ist ein deutlicher Vorteil gegenüber den rein wässrigen Verfahren und erleichtert die CIP-Reinigung

### Zu Schritt F) Separation

Die Separation soll anhand einiger Beispiele zur besseren Veranschaulichung nachfolgend erläutert werden.

### Beispiel:

B1) Ein kalt gepresster proteinhaltiger Kuchen, welcher bis zum Schritt F verarbeitet wird, weist nach seiner Verarbeitung folgende Phasen auf: 17% schwere Phase als Schalenanteil vom Zulauf mit 20 % der Kuchen-Proteine und 83% Oberlauf als Protein-Polyphenol-Öl-Phosphatid-Phase mit 80 % der Kuchenproteine
B2) Ein warm gepresster Kuchen, welcher bis zum Schritt F verarbeitet wird, weist nach seiner Verarbeitung folgende Phasen auf: 26% Schwere Phase als Schalenanteil vom Zulauf mit 30 % der Kuchen-Proteine und 74% Oberlauf als Protein-Polyphenol-Öl-Phosphatid-Phase mit 70 % der Kuchenproteine
B3) Ein heiß gepresster Kuchen, welcher in Schritt F verarbeitet wird, weist nach seiner Verarbeitung folgende Phasen auf: 30% Schwere Phase als Schalenanteil vom Zulauf mit 50 % der Kuchen-Proteine und 70% Oberlauf als Protein-Polyphenol-Öl-Phosphatid-Phase mit 50 % der Kuchenproteine.

### Zu Schritt G) - Proteinfällung

Aus dem Oberlauf (Oberlauf = leichte Phase) der Separation im vorangegangenen Schritt werden die Proteine durch pH-Verschiebung auf den Bereich von 4,5 bis ca. 7 ausgefällt. Die wasserunlöslichen Proteine, welche jedoch in wässriger Lösung quellbar sind, bilden zusammen mit den ausgefallenen Globulinen die Proteinfraktion des "Proteinquarks". Die Flüssigkeit in dieser Fraktion hat dieselbe Zusammensetzung wie die Flüssigkeit der Mittelphase (Oberlauf ohne Triglyceride). Da aber die Quarkphase nur 10-30 Gew.% des Zulaufes ausmacht, (mit einem relativ hohem Anteil an Trockenmasse, 15-25 Gew.% Trockensubstanz), sind in der Quarkphase mengenmäßig auch wesentlich weniger Polyphenole zu finden als in der Mittelphase, wenngleich die Konzentration der Polyphenole bezogen auf das Wasser dieselbe ist.

Damit ist eine Proteinphase aus wasserunlöslichen jedoch gequollenen Proteinen mit Globulinen verfügbar, die mit Polyphenolen abgereichert ist. Diese Kombination aus alkalisch-ethanolischem Milieu in den Schritten A-F gefolgt von einem saueralkoholischen Milieu zur Proteinfällung stellen sehr gute Bedingungen für eine Polyphenol-Extraktion dar. Überraschenderweise hat sich für Raps (Sinapin und Derivate) hier die Beobachtung für andere Polyphenole (Tyrosol und Derivate u.a,) aus anderen Bereichen wie der Verarbeitung von Oliven bestätigt, obwohl deutlich mehr reaktionsaktive Stoffe wie Proteine und Zucker in der Suspension enthalten sind.

Damit werden Verdünnungen wie in der Literatur beschrieben hinfällig, um auf gleichwertige Polyphenol Extraktionsraten des Wässrigen zu kommen (so etwa wiederum Kroll et al, "Rapssamenproteine - Struktur, Eigenschaften, Gewinnung und Modifizierung", Deutsche Lebensmittel-Rundschau, Heft 3, 2007, S. 109.

Da das reine Triglycerid aus der Flüssigkeit als leichte Phase verdrängt wird, kann der Restölgehalt im Protein-Endprodukt auf unter 15 Gew.%, auch unter 13 Gew.% bezogen auf Trockensubstanz gesenkt werden.

Da die Temperaturen während des gesamten Prozesses <= 50 °C betragen, kann auch von einem nativen Endprodukt gesprochen werden.

Vorteilhaft ist ein Scheren des weiter zu verarbeitenden Breis vor der Phasentrennung des Schrittes H (vor der Ölabtrennung) und nach Schritt F) oder G) des Anspruchs 1 zur Verbesserung der Verdrängungsextraktion. Dieses Scheren kann mit einer Schervorrichtung, wie z.B. einem Homogenisator oder einem Intensivmischer durchgeführt werden, um derart noch mehr Öl zu gewinnen.

Das Scheren mit einer Schervorrichtung kann im kontinuierlichen Prozess durchgeführt werden. Insgesamt wird vorzugsweise ein kontinuierlicher Prozess realisiert.

Zu Schritt H) - Separation der Proteine als Quark mittels Dekanter oder Separator Zur Erhöhung der Reinheit kann der Proteinquark gewaschen werden. Der Quark kann anschließend zu einem Pulver getrocknet werden.
Zu Schritt I) - Isolierung der Sinapinsäure, des Sinapins oder eines Derivates der Sinapinsäure

Anschließend erfolgt eine Gewinnung von Sinapinsäure mittels einer Extraktion und ggf. ein Gewinnen einer Albuminphase (letzteres beispielsweise durch Filtration).
Kurzgefasst wird auch ein vorteilhaftes Verfahren zur Gewinnung von Proteinen aus nativen Stoffgemengen geschaffen, mit folgenden Schritten:
A) Bereitstellen eines nativen Stoffgemenge aus Saaten mit harten, zerbrechbaren Schalen,
B) Aufbrechen bzw. Grob-Zerkleinern des Stoffgemenges, um jedenfalls die Schalen aufzubrechen, ohne sie zu fein zu dispergieren. Vorzugsweise soll die Größe der zerkleinerten Schalenanteile in einer granulometrischen Verteilung nach Art zwischen 100 und 2000 µm liegen. insbesondere mit einem Maximum zwischen 300 µm und 900 µm, insbesondere bei ca. 600 µm, jeweils bei einer relativen Häufigkeit von mehr als 5%);
C) Dispergieren des zerkleinerten aufgebrochenen Stoffgemenges aus Schritt A) oder B) mit Wasser oder einer wässrigen Lösung;
D) Einstellen des pH-Wertes des Breis (I) aus Schritt C) auf einen alkalischen Bereich von pH > 9, 5;
E) Zugeben des wasserlöslichen organischen Lösemittels Alkohol zu dem Brei aus Schritt C) im Anschluss an das Einstellen des pH-Wertes des Breis im Schritt D;
F) Abtrennen einer Feststoffphase, welche den überwiegenden Anteil der Schalen aufweist, vorzugsweise in einer Zentrifuge im Zentrifugalfeld;
G) Verschieben des pH-Wertes des von Schalen befreiten Breis aus Schritt F) in den pH-Bereich von pH = 4,5 bis pH = 7,2, und
H) Trennen des schalenfreien Breis, dessen pH-Wert wieder ins Saure verschoben worden ist, - vorzugweise in einer Zentrifuge, insbesondere in wenigstens einem Dekanter - in einem oder zwei Schritten in folgende drei Wertstoffphasen: ölhaltige Phase mit Triglyceringehalt; wässrige Phase mit Albumingehalt und Proteinkonzentratphase (Proteinquark).

Wenn der Ölgehalt im Rohmaterial sehr gering ist, entfällt die Ölphase und es entstehen nur zwei Phasen, die wässrige Albuminphase und die Proteinkonzentratphase

Es ist weiter vorteilhaft, wenn die Quarkphase getrocknet wird. Hier ist es vorteilhaft, noch vorhandenen Alkohol aus dem Quark zu verdampfen, vorzugsweise unter Vakuum, um die Temperatur niedrig zu halten und dann den alkoholfreien, wässrigen Quark zu einem Pulver zu trocknen. Hierzu sind beispielsweise eine Trocknung und eine Mahlung vorteilhaft; für dessen Umsetzung in einem Gerät der Mahltrockner geeignet ist. Derart wird ein lagerstabiles, leicht handhabbares sowie transportierbares Produkt geschaffen.

Die positiven Eigenschaften können anhand einer im Versuch gewonnenen Proteinphase veranschaulicht werden:
Versuch 1 (zu den Schritten A- -F): Versuchsansatz: Es wurden 95 kg Stadtwasser+ 23 kg Rapssaat (Warmpressung) in Rührvorlage abgefüllt und auf 40 °C erhitzt (Schritte A und C).

Anschließend wurde dieses Produkt-/Wassergemisch mittels einer Monopumpe und eines Fristam-Mischers bei 1000 l/h ca. 8 min im Kreislauf gefahren (pH = 6,2). Sodann erfolgt eine Zugabe von 4,1 l 10%iger NAOH und die Einstellung auf einen pH = 10 (Schritt D).

Anschließend wurde 15 min. ohne Fristam Mischer bei 1000 l/h im Kreislauf gefahren und gerührt. Dann erfolgt eine Zugabe von 14,2 kg Ethanol (Schritt E in einem oder mehreren Teilschritten) mittels Schlauchpumpe direkt in den Kreislauf der Monopumpe. Verweilzeit: vorzugsweise 10 - 50 min. Nach ca. 50 min Verweilzeit wird nochmals 2 kg Ethanol in 11 kg Wasser vermischt und in einen Rührwerksbehälter gegeben. 10 min Verweilzeit. Diese Suspension wird zentrifugal getrennt (Schritt F) Dabei beträgt die Ausbeute: 96,5 kg Klarphase, 34 kg Feststoffe. Die Schalen lassen sich somit leicht abtrennen.

Versuch 2: (A - F) Versuchsansatz: 116 kg Stadtwasser und 26 kg Rapssaat (Kaltpressung) in Rührvorlage wurden abgefüllt und auf 40 °C erhitzt. Anschließend wurde das Gemenge mittels Monopumpe und Fristam-Mischer bei 1000 l/h ca. 8 min im Kreislauf gefahren (pH = 5,8). Zugabe von 4,5 l 10%iger NaOH auf pH = 10. Anschließend wurde 15 min. ohne Fristam Mischer bei 1000 l/h im Kreislauf gefahren. Es erfolgte sodann eine Zugabe von 17,2 kg Ethanol mittels Schlauchpumpe direkt in den Kreislauf der Monopumpe. Es erfolgt eine10-minutige Verweilzeit. Danach wird separiert, um die Schalenfraktion abzutrennen. Die Ausbeute beträgt Ausbeute: 129,6 kg Klarphase, 26,5 kg Feststoffe. Die Schalen lassen sich somit leichter abtrennen.

Versuch 3: (Schritte G und H zu Versuch 1): 96,5 kg Klarphase vom Versuch l (pH-Anfangswert: 9,6) wurden mittels 0,8 l 25 % iger Salzsäure auf pH 5 verschoben, hier vorteilhaft bei 45°C) (Schritt G). Dieser Brei kann sodann zentrifugiert werden, wobei eine Proteinphase als schwere Phase bzw. Feststoffphase gewonnen wird (Schritt H). Ausbeute Klarphase: 64,3 kg, . Ausbeute Feststoffphase (Quarkartig) 9 kg Versuch 4 (Schritte G und H zu Versuch 2): 129,6 kg Klarphase vom Versuch II (pH-Anfangswert: 9,5) wurden mittels 1,2 l 25%iger Salzsäure auf pH 5 verschoben bei 45°C (Schritt G). Dieser Brei konnte sodann zentrifugiert werden, wobei eine Proteinphase als schwere Phase bzw. Feststoffphase gewonnen wurde (Schritt H). Ausbeute: 83 kg Klarphase, 29,5 kg Feststoff)Proteinphase). Hier ist die Ausbeute an Feststoffphase besonders groß.

Typischerweise weist ein nach Art der vorstehenden Versuche gewonnenen und sodann getrockneten Quark gewonnene Pulver Trockensubstanzgehalte von 5-9 % auf; in einem Muster hergestellt aus gewöhnlichem Presskuchen 5,35%. Die Proteingehalt beträgt ca. 60% Das Wasserbindevermögen wurde mit 1,8 +/- 0,2 ml H₂O auf 1 g Trockensubstanz des Proteinpulver ermittelt, das Ölbindevermögen mit 0,49 bis 0,63 g Öl je 1 g Trockensubstanz sowie die Emulgierbarkeit mit 700 bis 780 ml ÖL je 1 g Trockensubstanz des Proteinpulvers. Typische Werte für den Proteinlöslichkeitswert NSI sind 9 bis 16%. Die besten Ergebnisse werden mit dem kalt gepressten Presskuchen erzielt.

In weiteren Versuchen zeigte sich überraschend, dass auch die Rührtechnik bei dem Verfahren von Bedeutung ist, was sich insbesondere auf das Rühren des Schrittes C) (und ggf. D) und E)) des Anspruchs 1 bezieht.

So wurde kalt gepresster Rapspresskuchen in der im Anspruch 1 beschriebenen Verfahrensweise verarbeitet. Dabei wurde im Schritt C) einmal mit einem Flügel-Rührer gerührt und einmal mit einem Propeller-Rührer.

Der Flügel-Rührer sollte so betrieben werden, dass er möglichst wenige Scherkräfte beim Rühren erzeugt sondern eine im Wesentlichen gleichmäßig laminare Strömung.

Bei dem Propeller-Rührer im Sinne dieser Anmeldung sind Rührelemente auch außerhalb der Drehachse verbunden, so durch eine Scheibe oder einen Ring oder in Ihrer Nähe sind Elemente wie eine offene Glocke über den Propellerelementen vorhanden. Sie erzeugen daher beim Rühren eine relativ turbulente Strömung und üben höhere Scherkräfte auf das Produkt aus.

Flügel-Rührer sind also solche, die im Wesentlichen eine laminare Strömung beim Rühren erzeugen, die relativ lange Flügel aufweisen und die bei geringer Drehzahl betrieben werden. Ein Ring oder eine Scheibe oder dgl. am Außenumfang der Flügel oder in deren Nähe (nach Art eines offenen Käfigs oder einer offenen Glocke um die Flügel) ist in der Regel nicht vorhanden.

Bei den weiteren Versuchen erfolgte nach den Schritten D) und E) - vorzugsweise unter weiterem Rühren mit dem Flügel-Rührer oder dem Propeller-Rührer - das Abtrennen der schalenhaltigen Feststoffphase gemäß Schritt F).

Die Flüssigphase nach der Schalenabtrennung aus einer Suspension aus Rapspresskuchen, der 13,4 % Öl, 31,4 % Protein und 55,2% Sonstiges enthielt (wie Zellulose, Polyphenole, Saccharide etc.), war bei der Verwendung des Flügel-Rührers zum Rühren in Schritt C) und ggf. D) und E) deutlich proteinreicher als bei Verwendung eines Propeller-Rührers. Ca. 75% der Proteine des Kuchens wurden in diesem Oberlauf gefunden, dessen Trockenmasse sich aus 52,3 % Protein, 13,0 % Öl und ca. 34% Sonstigem zusammensetzt. Im Gegensatz wurden nur 62,5% der Proteine des Kuchens in dem vergleichbaren Oberlauf gefunden, wenn ein Propeller-Rührer eingesetzt wurde. Für diesen Fall hatte die Oberlauf-Trockenmasse nur ca. 37% Protein, ca., 14,7 % Öl sowie 48,0 sonstige Inhaltsstoffe.

Auch optisch zeigten sich überraschend deutliche Unterschiede. Die Schalenfraktion der Schleuderprobe aus der Suspension unter Verwendung des Flügel-Rührers sah deutlich marmorierter aus. Es wurde hierbei nur 42% % der Trockenmasse als Oberlauf abgetrennt, beim Propeller-Rührer betrug der Anteil der abgetrennten Trockenmasse 50%.

Es konnten darüber hinaus noch weitere vorteilhafte Verfahrensvarianten gefunden werden.

So verursacht eine hohe Alkohol-, insbesondere Ethanolkonzentration einen hohen Öl-Gehalt im "Globulin-Quark". Besonders vorteilhaft ist es im Schritt E) daher, wenn die Alkoholkonzentration kleiner als 20 Vol.% ist, insbesondere 13 bis 18 Vol.% beträgt, besonders vorzugsweise 15 Vol% ist.

Eine zu lange Reaktionszeit bei pH 10 (über Nacht) verursacht ebenfalls hohe Ölgehalte im Globulin-Quark. Eher niedrigere Temperaturen, insbesondere unter 43°C, wirken sich vorteilhaft bei der Globulinfällung bzw. -abtrennung aus und bewirken höhere Proteingehalte im Quark.

Als besonders vorteilhaft erscheinen weiter eine oder mehrere folgender weiterer Maßnahmen: Die Verwendung frischen Materials beim Pressen des Öls; ein kaltes Pressen des Öls und ein schonendes Rühren mit einem Flügel-Rührer (im Schritt C), wobei das Material möglichst wenig geschert werden gar gemahlen werden sollte. Zudem ist ein vorteilhafter Alkohol-, insbesondere Ethanolgehalt von weniger als 20% besonders vorteilhaft, da sich ansonsten ein höherer Ölgehalt im Quark ergibt.

Zur Sinapinsäuregewinnung des Schrittes I) wurden ebenfalls Versuche durchgeführt.

So hat sich gezeigt, dass bei einer Vorbehandlung der Schritte C), D) und E) die Sinapinsäure in der "Wasserphase" angereichert wird. Sie ist damit durch Extraktion mit einem geeigneten Extraktionsmittel gewinnbar, und zwar entweder nach dem Schritt H) oder schon nach dem Schritt F).

Dabei hat allerdings auch die Wahl des Ausgangsmaterials einen Einfluss auf die Menge der gewinnbaren Sinapinsäure.

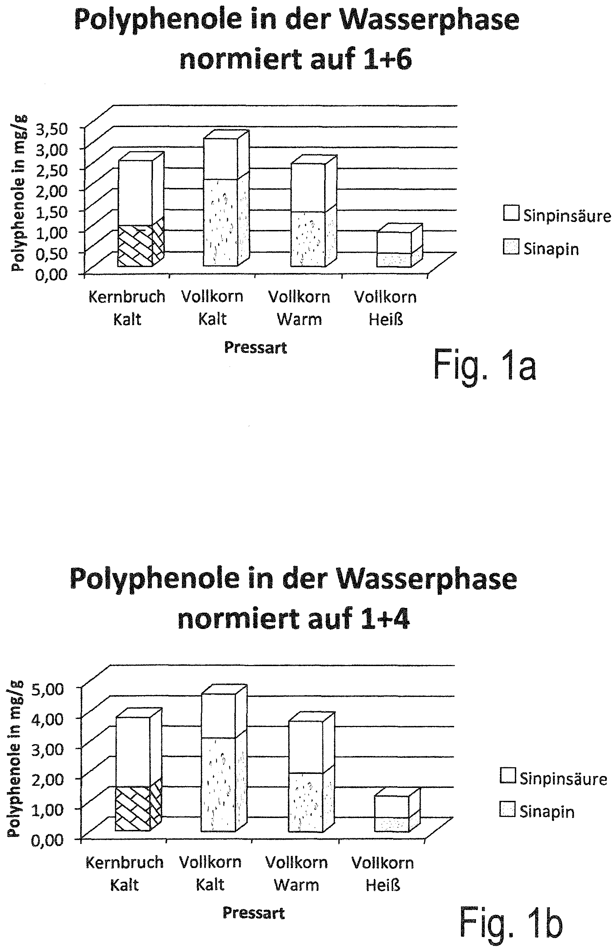

Hierzu sei auf die Diagramme der beiliegenden Fig. 1a und b hingewiesen. Bei den einzelnen Versuchen wurden unterschiedliche Ansätze aus verschiedenem Rohmaterial bzw. Ausgangsmaterial plus Wasser gewählt, wobei die Proben zwar verschiedene Mengen hatten, dies wurde aber normiert bzw. geeignet umgerechnet.

Die beiden Diagramme zeigen, dass der Polyphenolgehalt in der wässrigen Phase auf mehr als das 4-fache ansteigen kann, wenn als Ausgangsmaterial "kalt gepresster Rapspresskuchen" anstelle von "heißgepresstem Rapspresskuchen" genutzt wird. Die Verwendung des frischen Materials ist auch insoweit vorteilhaft. Denn genauso wie der Polyphenolanteil in der Wasserphase angereichert wird, wird er in der Proteinquarkphase abgereichert. So wird bei einem heiß gepressten Kuchen der Anteil von 9,4 % Polyphenolen (Trockensubstanz "TS" im Rohmaterial) auf 5,6 Gew.% TS im Proteinquark oder Quarkpulver abgereichert bzw. beim kalt gepressten Kuchen von 18,6 Gew.% TS auf 10,1 Gew.% TS im Quarkpulver. Somit ist diese Konzentration der Polyphenole bezogen auf die Trockenmasse im Feststoff nur noch etwa halb so groß wie im Ausgangsmaterial.

Damit ist eine Proteinphase aus wasserunlöslichen jedoch gequollenen Proteinen mit Globulinen verfügbar, die in Hinsicht auf den Polyphenolgehalt abgereichert worden ist. Es verbleiben in der Wasserphase ca. 55-55 Gew.% der Polyphenole in folgenden Konzentrationen:

| Kuchenart | Verdünnung beim Verfahren Anteil Wasser bezogen auf 1 Anteil Kuchen | PP in der Wasserphase (mg) | PP in der Wasserphase normiert auf eine Verdünnung 1 Teil Saat + 6 Teile Fluid |
|---|---|---|---|
| Kalt | 4,5 | 3976 | 2982 |
| Warm | 4,2 | 3183 | 2228 |
| Heiß | 6,0 | 1053 | 1058 |

Folgende Einflussfaktoren sollten bei der Verarbeitung beachtet werden: Beim Heißpressen werden Polyphenole (PP) abgebaut. Es ist gemessen worden, dass der PP-Gehalt bei kaltgepresster Saat bei 18 mg/g lag, bei heiß gepresster Saat jedoch bei 8,8 mg/g. aus der Literatur sind ähnliche Werte bekannt (6,2 mg/g bei Jeroch et al. 1999). Neben der Reduzierung der Polyphenole im Rohmaterial geht ein Entestern des Sinapins zu Sinapinsäure vonstatten.

Durch das Kaltpressen sind nach dem o.g. Verfahren die Polyphenole mengenmäßig am Stärksten bei der Kaltpressung in die Wasserphase bzw. präziser "Polphenol-Albumin-Phase" des Schrittes H) überführt. Sie liegen infolge der alkalischen Vorbehandlung (+Temperatur und EtOH) im Wesentlichen als Sinapinsäure oder Salze der Sinapinsäure vor und nicht mehr als Sinapin und noch nicht als Canolol.

Nunmehr ist es vorteilhaft, mit einem Extraktionsmittel, insbesondere Ethylacetat, die Sinapinsäure in einem Schritt I) aus der wässrigen Phase zu extrahieren. Dafür wurde die Polphenol-Albumin-Phase mit Ethylacetat oder alternativ mit Pentanol gewaschen
a) mit 1/1 (PP-Albumin Phase/Ethylacetat)
b) mit 1/2 (PP-Albumin Phase/Ethylacetat)
c) mit 1/1 (PP-Albumin Phase/Pentanol)
d) mit 1/2 (PP-Albumin-Phase/Pentanol)

Die einmal extrahierte Polyphenolphase als Wertstoffphase der Stufen a) bis d) wurde jeweils nochmals gewaschen (2.Extraktion. Dies wurde mit dem originalem pH von 5,2 bis 5,4 gemacht und nochmals bei pH 4 wiederholt. Derart ergab sich folgende Ergebnistabelle:

| | Extraktionsrate in Gew.% bei pH 5,2 | Extraktionsrate bei pH 5,2 und 2. Stufe | Extraktionsrate bei pH 4 | Extraktionsrate bei pH 4 und 2. Stufe |
|---|---|---|---|---|
| a Ethylacetat | 74,6 | 78,2 | 72,5 | 90,8 |
| b Ethylacetat 2.Wäsche | 57,8 | 77,0 | 82,7 | 91,1 |
| c Pentanol | 74,6 | 84,8 | 85,6 | 87,4 |
| d Pentanol 2.Wäsche | 61,1 | 80,7 | 85,5 | 92,1 |

Als besonders vorteilhaft erscheint eine Extraktion mit Ethylacetat. Dieses wird wiederum vorteilhaft im Verhältnis 1/0,5 bis 1/3 im wässrigen zugesetzt (Mengenverhältnis: PP-Albumin-Phase/Ethylacetat). Das Ethylacetat kann am Ende abgedampft werden.

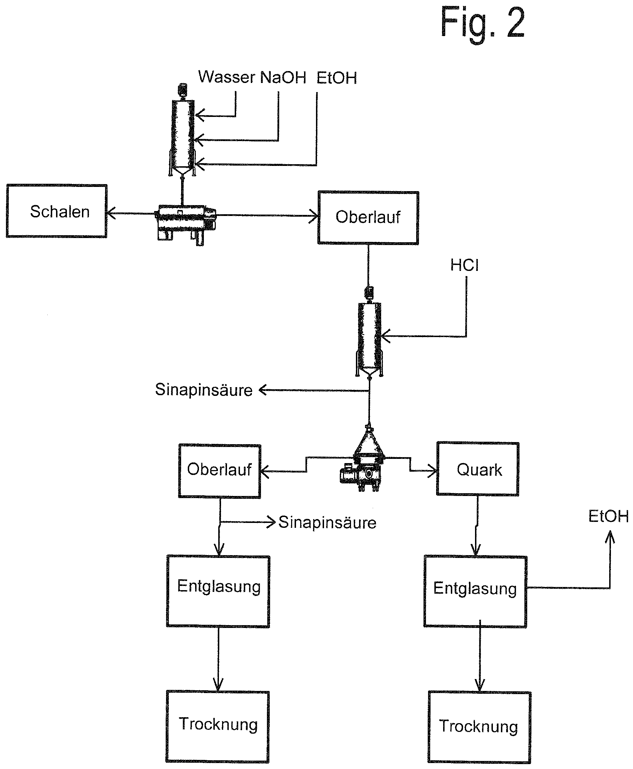

Die vorbeschriebenen Schritte sind nochmals anschaulich in Fig. 2 dargestellt.

## Patentansprüche

1. Verfahren zur Gewinnung von Sinapinsäure und/oder einem Salz der Sinapinsäure aus einem nativen Stoffgemenge, mit folgenden Schritten:
- Schritt A): Bereitstellen eines nativen Stoffgemenges aus Saaten mit harten, zerbrechbaren Schalen, insbesondere aus Saaten/Früchten von Kreuzblütengewächsen (Brassicaceae), insbesondere von Rapsfrüchten als Stoffgemenge aus den vollständigen Saaten oder aus bereits (teil-) entölten Saaten, insbesondere als Presskuchen, der bei einem Abpressen von Öl insbesondere mit einer Presse als Rückstand der Ölgewinnung verbleibt;
- Schritt B): sofern das Stoffgemenge aus Schritt A noch nicht zerkleinert ist: Zerkleinern des Stoffgemenges, wobei ggf. die Schalen aufgebrochen werden;
- Schritt C): Dispergieren des zerkleinerten Stoffgemenges aus Schritt A) oder B) mit Wasser, wobei auf einen Teil zerkleinertes Stoffgemenge vorzugsweise bis zu maximal 8, besonders vorzugsweise bis zu maximal 6, insbesondere bis zu maximal 5 Teile Wasser zugegeben werden und wobei das Wasser und das zerkleinerte Stoffgemenge gerührt werden, so dass sich ein fließfähiger Brei bzw. eine Dispersion ergibt;
- Schritt D): Einstellen des pH-Wertes des Breis aus Schritt C) in einen alkalischen Bereich pH > 9, 5;
- Schritt E): Zugeben eines wasserlöslichen organischen Lösemittels, vorzugsweise von Ethanol, insbesondere in mit Wasser verdünnter Form, zu dem Brei aus Schritt D) im Anschluss an das Einstellen des pH-Wertes des Breis im Schritt D; insbesondere derart, dass eine Alkoholkonzentration erreicht wird, die kleiner als 30% ist, um die Schalen vom Endosperm der Saaten/Früchte zu lösen;
- Schritt F): Abtrennen einer Feststoffphase, welche ggf. den überwiegenden Anteil der Schalen aufweist, vorzugsweise in einer Zentrifuge im Zentrifugalfeld;
- Schritt G): Verschieben des pH-Wertes des ggf. von Schalen befreiten Breis aus Schritt F) in den pH-Bereich von pH = 4,5 bis pH = 7,2; und
- Schritt H): Trennen des schalenfreien Breis, dessen pH-Wert in Schritt G) ins Saure verschoben worden ist, vorzugweise in einer Zentrifuge, insbesondere in wenigstens einem Dekanter oder einem Separator, in mehrere Phasen, wobei zumindest eine dieser Phasen eine Polyphenol-Albumin-Flüssigkeitsphase ist;
- Schritt I): Isolieren von Sinapinsäure und/oder eines Salzes der Sinapinsäure aus dem schalenfreien Brei des Schrittes F) oder G) oder aus der Polyphenol-Albumin-Flüssigkeitsphase des Schrittes H) direkt oder nach einem Durchlaufen weiterer Zwischenschritte, durch eine Extraktion mit einem Extraktionsmittel.

2. Verfahren nach Anspruch 1, **dadurch gekennzeichnet, dass** im Schritt H) folgende Phasentrennung in einem oder zwei Schritten, vorzugsweise in einer Zentrifuge, insbesondere in einem Dekanter oder Separator, vorgenommen wird:
- ölhaltige Phase mit Triglyceringehalt;
- wässrige Phase mit Albumin und Sinapinsäuregehalt; und
- ggf. eine dritte Phase mit einem weiteren Wertprodukt;

3. Verfahren nach Anspruch 1 oder 2, **dadurch gekennzeichnet, dass** im Schritt H) folgende Phasentrennung in einem oder zwei Schritten, vorzugsweise in einer Zentrifuge, insbesondere in einem Dekanter oder Separator, in zwei Wertstoffphasen vorgenommen wird, mit zumindest einer wässrigen Phase mit Albumingehalt und Sinapinsäuregehalt und Restölgehalt.

4. Verfahren nach Anspruch 1, 2 oder 3, **dadurch gekennzeichnet, dass** als das Extraktionsmittel Ethylacetat verwendet wird.

5. Verfahren nach Anspruch 1, 2 oder 3, **dadurch gekennzeichnet, dass** als das Extraktionsmittel Pentanol verwendet wird.

6. Verfahren nach einem der vorstehenden Ansprüche, **dadurch gekennzeichnet, dass** eine mehrstufige Extraktion vorgenommen wird.

7. Verfahren nach einem der vorstehenden Ansprüche, **dadurch gekennzeichnet, dass** das Extraktionsmittel abgedampft wird.

8. Verfahren nach einem der vorstehenden Ansprüche, **dadurch gekennzeichnet, dass** als das Stoffgemenge/Ausgangsmaterial "kurz zuvor hergestelltes Zwischenprodukt" verarbeitet wird , d.h. nach der Vorstufe sind nicht mehr als 31 Tage vergangen.

9. Verfahren nach Anspruch 8, **dadurch gekennzeichnet, dass** als das Stoffgemenge/Ausgangsmaterial "frisches Zwischenprodukt" verarbeitet wird, d.h. nach der Vorstufe dürfen nicht mehr als 3 Tage vergangen sein, vorzugsweise sogar nur weniger als 48 Stunden, insbesondere weniger als 24 Stunden.

10. Verfahren nach einem der vorstehenden Ansprüche, **dadurch gekennzeichnet, dass** als das Stoffgemenge in Schritt A kalt gepressten Material, insbesondere ein kalt gepresster Rapspresskuchen verwendet wird, der bei einer Temperatur kleiner als 70°C, besonders vorzugsweise sogar kleiner als 60°C, gepresst worden ist.

11. Verfahren nach einem der vorstehenden Ansprüche, **dadurch gekennzeichnet, dass** einer oder mehrere der Abtrennungsschritte in einem 3-Phasendekanter oder in zumindest zwei Schritten in 2-Phasendekantern erfolgt/erfolgen.

12. Verfahren nach einem der vorstehenden Ansprüche, **dadurch gekennzeichnet, dass** einer oder mehrere der Abtrennungsschritte in einem Düsenseparator erfolgt/erfolgen.

13. Verfahren nach einem der vorhergehenden Ansprüche, **dadurch gekennzeichnet, dass** das wasserlösliche organische Lösemittel ein linearer aliphatischer Alkohol ist.

14. Verfahren nach einem der vorhergehenden Ansprüchen, **dadurch gekennzeichnet, dass** der Gehalt an wasserlöslichem organischen Lösemittel im wässrigen Anteil des dem Breis (I) nach dem Zugeben des wasserlöslichen organischen Lösemittels weniger als 45 Vol. %, vorzugsweise weniger als 30 Vol % und besonders vorzugsweise weniger als 15 Vol %, beträgt.

15. Verfahren nach einem der vorhergehenden Ansprüche, **dadurch gekennzeichnet, dass** die Temperatur während sämtlicher Verfahrensschritte, wozu nicht das Pressen zur Erzeugen des Presskuchens gehört, unterhalb von 60 ° C liegt.

16. Verfahren nach einem der vorhergehenden Ansprüche, **dadurch gekennzeichnet, dass** die Temperatur während sämtlicher Verfahrensschritte, wozu nicht das dem Verfahren vorangehende Pressen zur Erzeugen eines Presskuchens als Ausgangsmaterial gehört, unterhalb von 50 ° C liegt.

## Claims

1. Method for obtaining sinapic acid and/or a salt of sinapic acid from a native material mixture comprising the following steps:
- step A): providing a native material mixture of seeds having hard frangible husks, in particular of seeds/fruits of crucifers (Brassicaceae), in particular of oil seed rape fruits as material mixture from the complete seeds or from previously (partially) deoiled seeds, in particular as press cake which remains when oil is pressed off, in particular using a press, as residue of obtaining oil;
- step B): if the material mixture of step A is not yet comminuted: comminuting the material mixture, wherein, optionally, the husks are broken open;
- step C): dispersing the comminuted material mixture of step A) or B) with water, wherein, to one part of comminuted material mixture, preferably up to a maximum of 8, particularly preferably up to a maximum of 6, in particular up to a maximum of 5, parts of water are added, and wherein the water and the comminuted material mixture are agitated such that a free-flowing pulp or a dispersion results;
- step D): setting the pH of the pulp from step C) to an alkaline range pH > 9.5;
- step E): adding a water-soluble organic solvent, preferably ethanol, in particular in water-diluted form, to the pulp from step D) subsequently to the setting of the pH of the pulp in step D; in particular in such a manner that an alcohol concentration is achieved which is less than 30%, in order to detach the husks from the endosperm of the seeds/fruits;
- step F): separating off a solids phase which may have the predominant fraction of the husks, preferably in a centrifuge in the centrifugal field;
- step G): shifting the pH of the pulp which is optionally freed from husks from step F) to the pH range from pH = 4.5 to pH = 7.2; and
- step H): separating the husk-free pulp, the pH of which has been shifted to the acidic range in step G), preferably in a centrifuge, in particular in at least one decanter or a separator, into a plurality of phases, wherein at least one of said phases is a polyphenol-albumin liquid phase;
- step I): isolating sinapic acid and/or a salt of sinapic acid from the husk-free pulp of step F) or G) or from the polyphenol-albumin liquid phase of step H) directly or after it passes through further intermediate steps, by an extraction with an extraction medium.

2. The method as claimed in claim 1, **characterized in that** phase separation following in step H) is performed in one or two steps, preferably in a centrifuge, in particular in a decanter or separator:
- oil-containing phase having a triglycerol content;
- aqueous phase having albumin and sinapic acid content; and
- optionally a third phase having a further product of value.

3. The method as claimed in claim 1 or 2, **characterized in that** phase separation following in step H) is performed in one or two steps, preferably in a centrifuge, in particular in a decanter or separator, into two valuable material phases, having at least one aqueous phase having an albumin content and sinapic acid content and residual oil content.

4. The method as claimed in claim 1, 2 or 3, **characterized in that** the extraction medium used is ethyl acetate.

5. The method as claimed in claim 1, 2 or 3, **characterized in that** the extraction medium used is pentanol.

6. The method as claimed in one of the preceding claims, **characterized in that** a multistage extraction is performed.

7. The method as claimed in one of the preceding claims, **characterized in that** the extraction medium is vaporized.

8. The method as claimed in one of the preceding claims, **characterized in that** the material mixture/starting material processed is "intermediate produced shortly before", that is to say after the preliminary stage no more than 31 days have passed.

9. The method as claimed in claim 8, **characterized in that** the material mixture/starting material processed is "fresh intermediate", that is to say after the preliminary stage no more than 3 days must have passed, preferably even only fewer than 48 hours, in particular fewer than 24 hours.

10. The method as claimed in one of the preceding claims, **characterized in that** the material mixture used in step A is cold-pressed material, in particular a cold-pressed oil seed rape press cake which has been pressed at a temperature below 70°C, particularly preferably even below 60°C.

11. The method as claimed in one of the preceding claims, **characterized in that** one or more of the separation steps proceeds in a three-phase decanter or in at least two steps in two-phase decanters.

12. The method as claimed in one of the preceding claims, **characterized in that** one or more of the separation steps proceeds in a nozzle separator.

13. The method as claimed in one of the preceding claims, **characterized in that** the water-soluble organic solvent is a linear aliphatic alcohol.

14. The method as claimed in one of the preceding claims, **characterized in that** the content of water-soluble organic solvent in the aqueous fraction of the pulp (I) after the addition of the water-soluble organic solvent is less than 45% by volume, preferably less than 30% by volume, and particularly preferably less than 15% by volume.

15. The method as claimed in one of the preceding claims, **characterized in that** the temperature during all of the method steps, which does not include the pressing to generate the press cake, is below 60°C.

16. The method as claimed in one of the preceding claims, **characterized in that** the temperature during all of the method steps, which does not include the pressing that precedes the method and is for generating a press cake as starting material, is below 50°C.

## Revendications

1. Procédé pour l'extraction d'acide sinapique et/ou d'un sel d'acide sinapique à partir d'une quantité de matière native, comprenant les étapes suivantes :
- étape A) : préparation d'une quantité de matière native à partir de graines à coque dure et broyable, en particulier de graines ou fruits de crucifères (*Brassicaceae*), en particulier de gousses de colza sous forme de mélange de matières composé des graines entières ou de graines déjà (partiellement) déshuilées, en particulier de tourteau laissé par le pressage d'huile, en particulier avec une presse, et constituant un résidu de l'extraction d'huile ;
- étape B) : si le mélange de matière de l'étape A n'a pas encore été broyé, broyage du mélange de matière en ouvrant si nécessaire les coques ;
- étape C) : dispersion du mélange de matière de l'étape A) ou B) broyé avec de l'eau, en ajoutant de préférence jusqu'à 8 parts au maximum, en particulier jusqu'à 6 parts au maximum et tout spécialement jusqu'à 5 parts au maximum d'eau pour une part de mélange de matière broyé et en agitant l'eau et le mélange de matière broyé de façon à obtenir une bouillie fluide ou une dispersion ;
- étape D) : ajustement du pH de la bouillie de l'étape C) dans une plage de pH alcalin > 9,5 ;
- étape E) : ajout d'un solvant organique hydrosoluble, de préférence d'éthanol, en particulier sous une forme diluée avec de l'eau, à la bouillie de l'étape D) après l'ajustement du pH de la bouillie dans l'étape D, en particulier de façon à obtenir une concentration d'alcool inférieure à 30 % afin de décoller les coques de l'endosperme des graines/fruits ;
- étape F) : séparation d'une phase solide, qui contient éventuellement la majeure partie des coques, de préférence dans une centrifugeuse, dans le champ de centrifugation ;
- étape G) : décalage du pH de la bouillie de l'étape F) éventuellement débarrassée des coques dans la plage de pH = 4,5 à pH = 7,2 ; et
- étape H) : séparation en plusieurs phases de la bouillie débarrassée des coques dont le pH a été acidifié dans l'étape G), de préférence dans une centrifugeuse, en particulier dans au moins un décanteur ou un séparateur, au moins une de ces phases étant une phase liquide polyphénol-albumine ;
- étape I) : isolement de l'acide sinapique et/ou d'un sel d'acide sinapique à partir de la bouillie débarrassée des coques de l'étape F) ou G) ou de la phase liquide polyphénol-albumine de l'étape H), directement ou après d'autres étapes intermédiaires, par extraction avec un agent d'extraction.

2. Procédé selon la revendication 1, **caractérisé en ce que** dans l'étape H), les phases suivantes sont séparées en une ou deux étapes, de préférence dans une centrifugeuse, en particulier dans un décanteur ou un séparateur :
- phase huileuse contenant de la triglycérine,
- phase aqueuse contenant de l'albumine et de l'acide sinapique et
- éventuellement une troisième phase contenant un autre produit valorisable.

3. Procédé selon la revendication 1 ou 2, **caractérisé en ce que** dans l'étape H), les phases suivantes sont séparées en une ou deux étapes, de préférence dans une centrifugeuse, en particulier dans un décanteur ou un séparateur, en deux phases valorisables, avec au moins une phase aqueuse contenant de l'albumine et de l'acide sinapique et de l'huile résiduelle.

4. Procédé selon la revendication 1, 2 ou 3, **caractérisé en ce que** de l'acétate d'éthyle est utilisé comme agent d'extraction.

5. Procédé selon la revendication 1, 2 ou 3, **caractérisé en ce que** du pentanol est utilisé comme agent d'extraction.

6. Procédé selon l'une des revendications précédentes, **caractérisé en ce que** l'extraction est réalisée en plusieurs étapes.

7. Procédé selon l'une des revendications précédentes, **caractérisé en ce que** l'agent d'extraction est évaporé.

8. Procédé selon l'une des revendications précédentes, **caractérisé en ce que** le mélange de matière/la matière première mise en oeuvre est un « produit intermédiaire fabriqué récemment », c'est-à-dire pas plus de 31 jours avant l'étape préliminaire.

9. Procédé selon la revendication 8, **caractérisé en ce que** le mélange de matière/la matière première mise en oeuvre est un « produit frais », c'est-à-dire fabriqué pas plus de 3 jours avant l'étape préliminaire, de préférence moins de 48 heures avant, en particulier moins de 24 heures.

10. Procédé selon l'une des revendications précédentes, **caractérisé en ce que** le mélange de matière utilisé dans l'étape A) est une matière pressée à froid, en particulier un tourteau de soja pressé à froid, qui a été pressé à une température inférieure à 70 °C, de préférence inférieure à 60 °C.

11. Procédé selon une ou plusieurs des revendications précédentes, **caractérisé en ce qu'**une ou plusieurs des étapes de séparation se déroulent dans un décanteur à 3 phases ou au moins deux étapes dans un décanteur à 2 phases.

12. Procédé selon une ou plusieurs des revendications précédentes, **caractérisé en ce qu'**une ou plusieurs des étapes de séparation se déroulent dans un séparateur à buses.

13. Procédé selon l'une des revendications précédentes, **caractérisé en ce que** le solvant organique hydrosoluble est un alcool aliphatique.

14. Procédé selon l'une des revendications précédentes, **caractérisé en ce que** la teneur en solvant organique hydrosoluble dans la fraction aqueuse de la bouillie (I) après l'ajout du solvant organique hydrosoluble est inférieure à 45 % du volume, de préférence inférieure à 30 % du volume et en particulier inférieure à 15 % du volume.

15. Procédé selon une ou plusieurs des revendications précédentes, **caractérisé en ce que** la température pendant toutes les étapes de procédé, dont ne fait pas partie le pressage pour produire le tourteau, est inférieure à 60 °C.

16. Procédé selon une ou plusieurs des revendications précédentes, **caractérisé en ce que** la température pendant toutes les étapes de procédé, dont ne fait pas partie le pressage préalable au procédé pour la production du tourteau servant de matière première, est inférieure à 50 °C.
